# EUROPEAN PATENT SPECIFICATION

(11) **EP 1 931 265 B1**
(45) Date of publication and mention of the grant of the patent: **07.12.2011**
(21) Application number: 06825153.7
(22) Date of filing: 28.09.2006
(51) Int. Cl.: A61B 17/12, A61L 31/16, A61L 27/40, A61B 17/00, A61B 19/00

(54) **COATED VASO-OCCLUSION DEVICE**
BESCHICHTETE GEFÄSSVERSCHLUSSVORRICHTUNG
DISPOSITIF VASO-OCCLUSIF REVETU

(30) Priority: 30.09.2005 US 722360 P
(43) Date of publication of application: 18.06.2008
(60) Divisional application: 11161958.1
(73) Proprietor: Cook Medical Technologies LLC, Bloomington, IN 47402 (US)
(72) Inventor: BATES, Brian, L., Bloomington, IN 47401 (US)
(74) Representative: Garratt, Peter Douglas
(86) International application number: PCT/US2006/037595
(87) International publication number: WO 2007/041131

(56) References cited:
- EP-A- 1 543 849
- WO-A-03/009764
- WO-A-03/015640
- WO-A-2004/091712
- WO-A2-00/13624
- WO-A2-01/93920
- US-A- 5 733 329
- US-A1- 2002 082 620

## Description

### Technical Field

Aneurysms present a potentially life-threatening problem. An aneurysm is the result of a weak area in a vessel wall, resulting in bulging in the weak area at a particular site in the vessel wall. Untreated aneurysms stand the risk of rupturing, which can result in a stroke or even death.

### Background of the Invention

Endovascular embolization provides an alternative, non-surgical treatment for aneurysms or for other medical situations were vascular occlusion is desired. Typically, soft platinum coils (or stainless steel coils having increased radial strength) are deposited through a microcatheter into the catheter. The softness of the platinum minimizes ruptures and allows the coil to conform to the often irregular shape of the aneurysm or desired occlusion site. Generally, an average of 5-6 coils is used to pack an aneurysm. The goal of this treatment is prevent blood flow into the aneurysm sac by filling the aneurysm with coils. Preventing blood flow eliminates the risk of rupture. By reducing blood flow, hemostasis and the formation of clots/thrombi can occur within *e.g.*, the aneurysm or proximal to the aneurysm neck.

However, standard platinum embolization coils are biologically inert and are limited in their ability to promote thrombogenicity, or clotting. Even when a clot does form, however, it is susceptible to lysis. This can lead to an influx of blood resulting in rupture of the aneurysm. Moreover, platinum coils do not necessarily provide complete packing of the aneurysm lumen. Consequently, it is not uncommon for the aneurysm to re-canalize, enlarge and even rupture. In wider neck aneurysms, embolization coils have been found to migrate back to the parent vessel, which may result in occlusion of the parent vessel. Migration of embolization coils through the blood into other areas can be potentially dangerous. In view of the shortcomings associated with endovascular treatment of aneurysms, there is a need in the art for improved embolization coils conferring stability and maximum thrombogenicity. Document EP1543849A discloses a device according to the preamble of claim 1.

### Summary of the Invention

In one aspect, the present invention provides a vaso-occlusion device containing at least one occluding element, the occluding element including a polymeric material having a plurality of holes at least partially filled with a bioactive agent, where the bioactive agent promotes occlusion of a vessel in a subject and where the vaso-occlusion device is configured to occlude a vessel in the subject. Exemplary polymeric materials include THORALON, or other porous, polymeric materials supporting release of bioactive agents. The bioactive agent promotes thrombogenicity, inhibiting thrombolytic activity, or both. Exemplary bioactive agents include those that are thrombogenic, fibrogenic, angiogenic, antithrombolytic, antifibrinolytic, fibrin stabilizing, wound healing, fibroblast stimulatory, vascularization promoting, cell and/or tissue attachment promoting, extracellular matrix promoting, and/or combinations thereof. The bioactive agent may be a resorbable agent promoting extracellular matrix deposition and/or bioremodeling so as to promote stabilization and/or securement of the vaso-occlusion device in a vessel site of a subject.

The vaso-occlusion device includes an occluding element in the form of a space-filling structural element. Exemplary space-filling structural elements include coils, wires, detachable coils, detachable wires, filamentous elements, threads, multi-wired threads, synthetic fibers and/or combinations. Space-filling structural elements may assume any three-dimensional configuration sufficient for occluding a vessel. Accordingly, a vaso-occlusion device may include a plurality of space-filling structural elements, any one of which may be coiled, bunched up etc.

In another embodiment, the vaso-occlusion device may include an occlusion bag enclosing a space-filling structural element or space-filling material, such that the enclosed occlusion bag is expanded to occlude a vessel in a subject. Exemplary space-filling materials include resorbable agents, including reconstituted or naturally-derived collagenous material, extracellular matrix material (ECM), small intestinal submucosal material (SIS) and the like.

A method for occluding a vessel in a patient is described in which a vaso-occlusion device of the present invention is positioned in a vessel of a patient to occlude the vessel and/or promote thrombus formation.

It is believed that use of the drug-embedded vaso-occlusion device of the present invention promotes clotting, aneurysm retraction and/or aneurysm scarring, thereby providing an improvement over standard vaso-occlusion devices of the prior art.

Other features and advantages of the invention will be, or will become, apparent to one with skill in the art upon examination of the following figures and detailed description. It is intended that all such additional systems, features and advantages are included within this description, are within the scope of the invention, and are protected by the following claims.

### Brief Description of the Drawing

FIG. 1A depicts a vaso-occlusion device as a space-filling structural element in the form of a bunched up thread or coil coated with a porous, polymeric material. FIG. 1B depicts a cross-section of a portion of the space-filling member of FIG. 1A coated with the porous, polymeric material.

FIG. 2 depicts a cross-section of a vaso-occlusion device having a circular occlusion bag enclosing a plurality of space-filling structural elements, the occlusion bag coated with a porous, polymeric material.

### Detailed Description

In order to provide a clear and consistent understanding of the specification and claims, the following definitions are provided.

As used herein, the term "vessel" is defined as including any bodily canal, conduit, duct or passageway, including but not limited to aneurysms, blood vessels, bile ducts, esophagus, trachea, ureter and urethra.

The term "vaso-occlusion device" refers to a composite structure configured to occlude a vessel in a subject. The vaso-occlusion device includes one or more occluding elements.

The term "occluding element" refers to a component of the vaso-occlusion device. The occluding element may include an occlusion bag, space-filling structural element and/or space-filling material.

The term "space-filling structural element" refers to a structural element having a three-dimensional shape configured for occluding a vessel. The space-filling structural element does not imply any particular secondary structure or shape. Exemplary space-filling structural elements include occlusion bags, coils, wires, detachable coils, detachable wires, multi-wired strands, cables, braids, polymeric sheet materials, fabrics, textiles, and combinations thereof.

The term "space-filling material" refers to a type of material filling an occlusion bag. Filling of the occlusion bag by the space-filling material is primarily determined by the shape of the occlusion bag. The space-filling material is not configured to any particular three-dimensional shape.

The term "polymeric material" refers to a polymeric sheet or polymeric coating material having a plurality of holes which is coated on at least one surface of an occluding element.

The term "sheet" means a monolithic layer of material. As used herein, the term "sheet" does not imply any particular shape, but includes flat layers, tubes, or other thin shaped objects. As used herein, the term "sheet" specifically includes textile materials formed from individual fibers, such as knitted or woven textiles or nonwoven textiles; and porous polymeric sheets, formed from polyesters, fluorinated polymers, polysiloxanes, polyurethanes, polyolefins, polyacrylonitrile, nylons, polyaramids and polysulfones. The term "polymeric sheet" means a monolithic layer of textile or porous polymer material. The term "porous sheet" means a cohesive layer of material containing spaces or holes.

The term "holes" means naturally-forming spaces within or between parts of the polymeric material. Holes may include interstices, pores, cavities, apertures, and spaces. The holes may include pores created by treatment of polymeric materials with particulate substances or pore forming agents, including inorganic salts. The holes may also include naturally resulting spaces formed from the joining of materials, such as textile fibers. Accordingly, textile materials may include holes as interstices between individual textile fibers and/or holes within individual textile fibers. The term "holes" does not include holes molded or drilled in, or otherwise directly imparted into materials by mechanical processes.

The term "bioactive agent" refers to a protein, peptide, peptidomimetic, organic molecule, synthetic molecule, drug, or synthetic polymer having at least one biological property selected from the group consisting of thrombogenic, fibrogenic, angiogenic, antithrombolytic, antifibrinolytic, fibrin stabilizing, wound healing, fibroblast stimulatory, vascularization promoting, cell and/or tissue attachment promoting, extracellular matrix promoting, and the like.

The term "thrombogenic fibrous material" refers to a synthetic and/or natural fibrous material having thrombogenic properties. Exemplary thrombogenic fibrous materials include, but are not limited to, DACRON, cotton, silk, wool, polyester thread and the like.

The term "resorbable agent" refers to a reconstituted or naturally-derived collagenous material, including extracellular matrix material, purified collagen, a native collagen containing tissue structure, such as tela submucosa tissue, and the like.

The term "extracellular matrix material" (ECM) refers to a collagenous tissue materials from extracellular matrix tissues. ECM materials include submucosa, renal capsule membrane, dermal collagen, dura mater, pericardium, fascia lata, serosa, peritoneum or basement membrane layers, including liver basement membrane.

The term "tela submucosa" refers to a natural collagen-containing submucosal tissue structure removed from a variety of sources including the alimentary, respiratory, intestinal, urinary or genital tracts of warm-blooded vertebrates. Tela submucosa material according to the present invention includes tunica submucosa, but may include additionally adjacent layers, such the lamina muscularis mucosa and the stratum compactum. Tela submucosa is a decellularized or acellular tissue, which means it is devoid of intact viable cells, although some cell components may remain in the tissue following purification from a natural source. Alternative embodiments (e.g., fluidized compositions etc.) include tela submucosa material expressly derived from a purified tela submucosal matrix structure. Tela submucosa materials according to the present disclosure are distinguished from collagen materials in other devices that do not retain their native submucosal structures or that were not prepared from purified submucosal starting materials first removed from a natural submucosal tissue source.

The term "small intestinal submucosa" (SIS) refers to a particular type of tela submucosal structure removed from a suitable small intestine source, such as pig.

The term "radiopaque" is defined as a non-toxic material that can be monitored or detected during injection into a mammalian subject by radiography or fluoroscopy. The radiopaque material may be either water soluble or water insoluble. Examples of water soluble radiopaque materials include metrizamide, iopamidol, iothalamate sodium, iodomide sodium, and meglumine. Examples of water insoluble radiopaque materials include tantalum, tantalum oxide, and barium sulfate, which are commercially available in the proper form for in vivo use. Other water insoluble radiopaque materials include, but are not limited to, gold, tungsten, stainless steel, and platinum.

In one aspect, the present invention provides a vaso-occlusion device containing one or more occluding elements, the occluding element(s) including a polymeric material having a plurality of holes at least partially filled with one or more bioactive agents, where the bioactive agent(s) promote occlusion of a vessel in a subject and where the vaso-occlusion device is configured to occlude a vessel in the subject. The bioactive agent may promote thrombogenicity, may inhibit thrombolytic activity, or both. The bioactive agent may be a resorbable agent promoting extracellular matrix deposition, bioremodeling, and/or stabilization and/or securement of the vaso-occlusion device in a vessel site of a subject. Exemplary resorbable agents include collagen-based materials or ECM-based structures, including e.g., small intestinal submucosal (SIS) structures.

The occluding element is a space-filling structural element configured to occlude a vessel in a subject. Exemplary space-filling structural elements include coils, wires, detachable coils, detachable wires, filamentous elements, threads, multi-wired threads, synthetic fibers, or combinations thereof.

The occluding element may also be in an occlusion bag containing a lumen enclosing one or more space-filling structural element(s) (as e.g., above) and/or space-filling material(s), such that the enclosed occlusion bag is expanded to occlude a vessel in a subject. Exemplary space-filling materials may include resorbable agents, such as Type I to Type XIV collagens; tela submucosal tissues, including small intestinal submucosal (SIS) tissues; fibrinogen; vitronectin; combinations and/or derivatives therefrom, and the like.

The vaso-occlusion device may further include one or more thrombogenic fibrous materials to promote embolization/thrombus formation. Exemplary thrombogenic fibrous material(s) may include Dacron, cotton, silk, wool, polyester thread, combinations and/or derivatives therefrom, and the like. Thrombogenic fibrous material(s) may be included in any part of the vaso-occlusion device, including the polymeric material, the space-filling structural elements or materials, the occlusion bag etc. Preferably, a thrombogenic fibrous material is included in or structurally linked to an outer surface of the vaso-occlusion device.

The vaso-occlusion device may further include at least one radiopaque and/or MRI compatible marker material to facilitate angiographic visualization of the vaso-occlusion device. The radiopaque and/or MRI-compatible marker material may be included in any part of the vaso-occlusion device, including the polymeric material, the space-filling structural elements or materials, the occlusion bag etc.

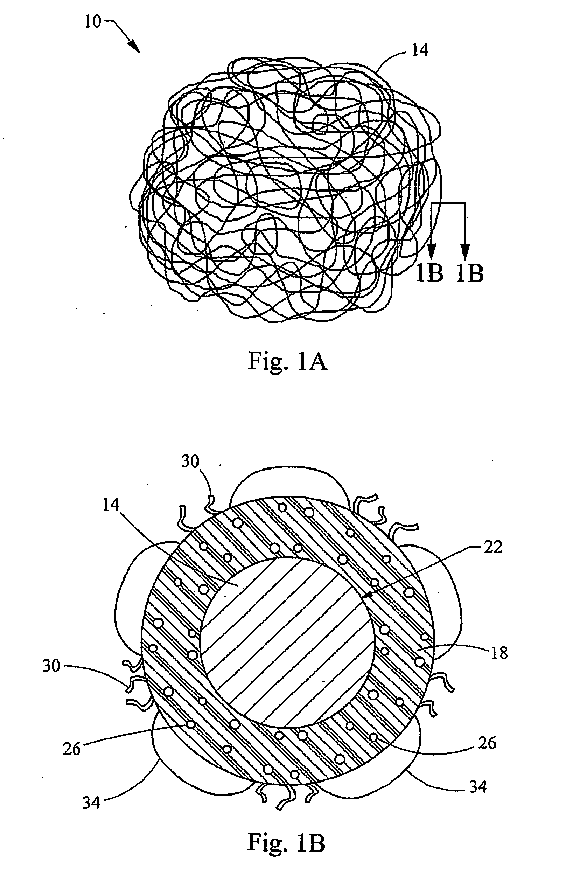

FIG. 1A depicts a representative vaso-occlusion device 10 in which the occluding element includes a coiled space-filling structural element 14 in the form of a bunched up coil or thread. FIG. 1B depicts a cross-section of a thread portion (hatched area) of the space-filling structural element 14 in FIG. 1A illustrating a surface 22 of the space-filling structural element 14 coated with the polymeric material 18 having a plurality of holes 26. The coated space-filling structural element 14 may be partially filled with at least one bioactive agent. The vaso-occlusion device 10 and/or space-filling structural element 14 may also include, substantially include, or be made from a polymeric material 18. The vaso-occlusion device 10 or polymeric material 18 may further include a thrombogenic fibrous material 30 to promote embolization/thrombus formation and/or a resorbable agent 34 to promote extracellular matrix deposition and bioremodeling, so as to stabilize and/or secure the vaso-occlusion device 10 in a vessel site of a subject. The thrombogenic fibrous material 30 and/or the resorbable agent 34 may be included in or structurally linked to the polymeric material 18 and/or to a surface 22 of the space-filling structural element 14 or vaso-occlusion device 10.

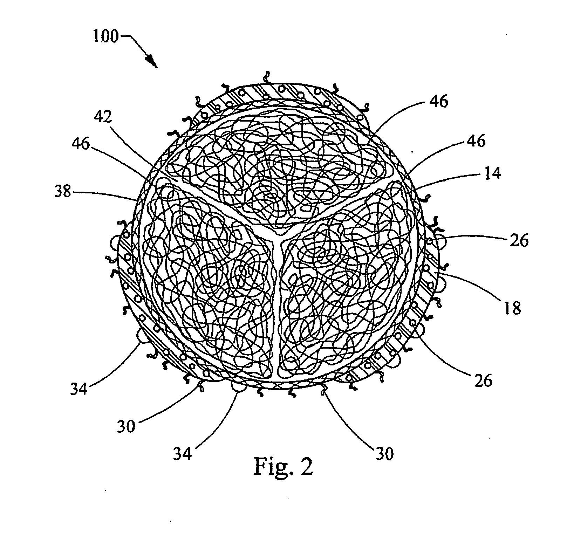

FIG. 2 depicts a cross-sectional view of a representative vaso-occlusion device 100 having a plurality of occluding elements, including an expandable occlusion bag 38 containing a lumen 42, and one or more space-filling structural elements 14 for occluding and/or anchoring the vaso-occlusion device 100 in a vessel, where the occlusion bag 38 is coated with a polymeric material 18 having a plurality of holes 26. The occlusion bag 38 may also be made from or substantially include the polymeric material 18. The occlusion bag 38 or the polymeric material 18 may further include or be structurally linked to a thrombogenic fibrous material 30 to promote embolization/thrombus formation. The vaso-occlusion device 100 may further include one or more resorbable agents 34 to promote extracellular matrix deposition and bioremodeling, so as to stabilize and/or secure the vaso-occlusion device 100 in a vessel site of a subject. The resorbable agents 34 may be included in or structurally linked to the occlusion bag 38 and/or the polymeric material 18. Alternatively, resorbable agents may be used as space-filling materials to expand the occlusion bag 38 (*i.e.,* in place of the space-filling structural elements).

The occlusion bag 38, space-filling structural element 14, or polymeric material 18 may further include or be structurally linked to a bioactive agent. For example, a bioactive agent may be impregnated in the polymeric material 18. One or more polymeric materials 18 may be additionally coated onto one or more surfaces 22 of a space-filling structural element 14 contained within the occlusion bag 38. In this case, the polymeric material 18 may contain a combination of bioactive agents, including resorbable agents 34, *etc*. Alternatively, space-filling material in the occlusion bag 38 may include in part or in whole a resorbable agent 34.

An occluding element may be made from natural or synthetic materials having a range of secondary shapes or space-filling materials suitable for expanding an occlusion bag 38 and/or occluding a vessel. The occluding element may include a space-filling structural element 14. Exemplary space-filling structural elements 14 include, but are not limited to, occlusion bags, coils, wires, detachable coils, detachable wires, multi-wired strands, cables, braids, polymeric sheet materials, fabrics, textiles, and the like. The space-filling material for expanding an occlusion bag 38 may include a natural material, including a resorbable agent or other space-filling materials suitable for occluding a vessel (natural or synthetic).

Occlusion bags 38 for use as occluding elements may be expanded in shape by any space-filling material suitable for expanding the occlusion bag 38 so as to promote occlusion at a given target site in a vessel. The occlusion bag 38 may be made from or include synthetic materials, such as nylon, fabric, textile, combinations thereof, and/or porous derivatives thereof. The occlusion bag 38 may also be made from natural materials, including resorbable agents, such as ECM or tela submucosa materials, and/or combinations and derivatives thereof.

At least one surface of the occlusion bag 38 is coated with at least one polymeric material 18 of the present invention. Accordingly, a polymeric material 18 is coated on an occlusion bag 38 made from natural or synthetic materials. Alternatively, the occlusion bag 38 may be made from or substantially include the polymeric material 18.

The occlusion bag 38 may accommodate any shape suitable for holding one or more space-filling structural elements or space-filling materials, including circular, spherical, cylindrical, oval and the like. The occlusion bag 38 may be filled with at least one occluding element and/or space-filling material suitable for expanding the occlusion bag 38 to occlude a vessel in a subject. Preferably, the size of the occlusion bag 38 may range from about 3 to 9 mm diameter. However, the size of the occlusion bag 38 may be larger or smaller depending on the size of the vessel 30 to be occluded.

Preferably, the space-filling materials used to expand the occlusion bags include natural materials, including resorbable agents, including ECM or tela submucosa materials, and/or combinations and derivatives thereof. Exemplary resorbable agents used as space-filling materials include, but are not limited to, Type I to Type XIV collagens, tela submucosal tissues, fibrinogen, vitronectin, and combinations and/or derivatives therefrom.

Embolization coils or wires for use as space-filling structural elements 14 are known in the art and may be made from various metal or metal alloy materials, containing platinum, stainless steel, gold; nickel-based alloys, such as NITINOL and INCONEL and may include other shape memory materials known in the art. Representative coils are disclosed in *e.g.*, U.S. Pat. No. 5,122,132 (Guglielmi et al.) and U.S. Pat. No. 5,334,210 (Gianturco). The coils or wires may be heat-set or pre-shaped to assume a desired shape consistent with expansion and occlusion of a vessel. Preferably, the embolization coils are designed for detachable delivery.

The diameter and length of a space-filling structural element 14 may range in size, depending on the nature and size of the vessel to be occluded. For example, coil lengths may range between about 2 cm and 20 cm; coil diameters may range from about 2 mm and 16 mm. An occlusion bag 38 may be filled with one or more space-filling structural elements 14 in the form of *e.g.*, a coil or a plurality of coils. A coil may comprise one or more helical loops, preferably at least 3-5 loops, and may be formed from a single wire or from a cable or braided structure of several wires. The size of the space-filling structural element 14 may range in size from about 3 to 9 mm. However, the size of the space-filling structural element 14 may be larger or smaller depending on the size of the vessel 30 to be occluded.

At least one surface of the vaso-occlusion device contains a polymeric material 18. One or more polymeric materials 18 may cover or coat part or all of the external surfaces in the vaso-occlusion device. A polymeric material 18 may include any porous, polymeric sheet or coating supporting release of bioactive agents and/or resorbable agents. A polymeric material 18 may be included with or coated onto any occluding element, including a space-filling structural element 14, space-filling material, occlusion bag 38, or combination thereof. Any part of the vaso-occlusion device may also be made from or made to include the polymeric material 18.

The polymeric material 18 includes a biocompatible polymeric sheet or coating containing a plurality of holes 26. Any polymer that may be formed into a porous material may be incorporated into (or coated onto) the vaso-occlusion device, provided the final porous material is biocompatible. The term "biocompatible" refers to a material that is substantially non-toxic in the *in vivo* environment of its intended use, and that is not substantially rejected by the patient's physiological system (i.e., is non-antigenic). This can be gauged by the ability of a material to pass the biocompatibility tests set forth in International Standards Organization (ISO) Standard No. 10993 and/or the U.S. Pharmacopeia (USP) 23 and/or the U.S. Food and Drug Administration (FDA) blue book memorandum No. G95-1, entitled "Use of International Standard ISO-10993, Biological Evaluation of Medical Devices Part-1: Evaluation and Testing." Typically, these tests measure a material's toxicity, infectivity, pyrogenicity, irritation potential, reactivity, hemolytic activity, carcinogenicity and/or immunogenicity. A biocompatible structure or material, when introduced into a majority of patients, will not cause a significantly adverse, long-lived or escalating biological reaction or response, and is distinguished from a mild, transient inflammation which typically accompanies surgery or implantation of foreign objects into a living organism.

The polymeric material 18 may include natural or synthetic materials including, but not limited to polyesters, such as polyethylene terephthalate, polylactide, polyglycolide and copolymers thereof; fluorinated polymers, such as polytetrafluoroethylene (PTFE), expanded PTFE and poly(vinylidene fluoride); polysiloxanes, including polydimethyl siloxane; and polyurethanes, including polyetherurethanes, polyurethane ureas, polyetherurethane ureas, polyurethanes containing carbonate linkages and polyurethanes containing siloxane segments. In addition, materials that are not inherently biocompatible may be subjected to surface modifications in order to render the materials biocompatible. Examples of surface modifications include graft polymerization of biocompatible polymers from the material surface, coating of the surface with a crosslinked biocompatible polymer, chemical modification with biocompatible functional groups, and immobilization of a compatibilizing agent such as heparin or other substances.

Polymers that can be formed into a porous sheets or coatings include polyolefins, polyacrylonitrile, nylons, polyaramids and polysulfones, in addition to polyesters, fluorinated polymers, polysiloxanes and polyurethanes as listed above. Preferably the polymeric material 18 includes a porous sheet or polymeric coating made of one or more polymers that do not require treatment or modification to be biocompatible. More preferably, the porous sheet or polymeric coating includes a biocompatible polyurethane. Examples of biocompatible polyurethanes include THORALON (THORATEC, Pleasanton, CA), BIOSPAN, BIONATE, ELASTHANE, PURSIL and CARBOSIL (POLYMER TECHNOLOGY GROUP, Berkeley, CA).

In a preferred embodiment, the porous polymeric material 18 includes or substantially includes a biocompatible polyurethane, such as THORALON, as described in U.S. Pat. Nos. 4,675,361 and 6,939,377. THORALON is a polyurethane urea base polymer (referred to as BPS-215) blended with a siloxane containing surface modifying additive (referred to as SMA-300). The concentration of the surface modifying additive may be in the range of 0.5% to 5% by weight of the base polymer.

The SMA-300 component (THORATEC) is a polyurethane containing polydimethylsiloxane as a soft segment and the reaction product of diphenylmethane diisocyanate (MDI) and 1,4-butanediol as a hard segment. A process for synthesizing SMA-300 is described, for example, in U.S. Pat. Nos. 4,861,830 and 4,675,361.

The BPS-215 component (THORATEC) is a segmented polyetherurethane urea containing a soft segment and a hard segment. The soft segment is made of polytetramethylene oxide (PTMO), and the hard segment is made from the reaction of 4,4'-diphenylmethane diisocyanate (MDI) and ethylene diamine (ED).

THORALON has been used in certain vascular applications and is characterized by thromboresistance, high tensile strength, low water absorption, low critical surface tension, and good flex life. THORALON is believed to be biostable and to be useful in vivo in long term blood contacting applications requiring biostability and leak resistance. Because of its flexibility, THORALON is useful in larger vessels, such as the abdominal aorta, where elasticity and compliance is beneficial.

THORALON can be manipulated to provide either porous or non-porous THORALON. Porous THORALON can be formed by mixing the polyetherurethane urea (BPS-215), the surface modifying additive (SMA-300) and a particulate substance in a solvent. The particulate may be any of a variety of different particulates or pore forming agents, including inorganic salts. Preferably the particulate is insoluble in the solvent. Examples of solvents include dimethyl formamide (DMF), tetrahydrofuran (THF), dimethyacetamide (DMAC), dimethyl sulfoxide (DMSO), or mixtures thereof. The composition can contain from about 5 wt% to about 40 wt% polymer, and different levels of polymer within the range can be used to fine tune the viscosity needed for a given process. The composition can contain less than about 5 wt% polymer for some spray application embodiments. The particulates can be mixed into the composition. For example, the mixing can be performed with a spinning blade mixer for about an hour under ambient pressure and in a temperature range of about 18 °C to about 27 °C. The entire composition can be cast as a sheet, or coated onto an article such as a mandrel or a mold. In one example, the composition can be dried to remove the solvent, and then the dried material can be soaked in distilled water to dissolve the particulates and leave pores in the material. In another example, the composition can be coagulated in a bath of distilled water. Since the polymer is insoluble in the water, it will rapidly solidify, trapping some or all of the particulates. The particulates can then dissolve from the polymer, leaving pores in the material. It may be desirable to use warm water for the extraction, for example water at a temperature of about 60 °C. The resulting void-to-volume ratio can be substantially equal to the ratio of salt volume to the volume of the polymer plus the salt. The resulting pore diameter can also be substantially equal to the diameter of the salt grains.

The porous polymeric sheet can have a void-to-volume ratio from about 0.40 to about 0.90. Preferably the void-to-volume ratio is from about 0.65 to about 0.80. The resulting void-to-volume ratio can be substantially equal to the ratio of salt volume to the volume of the polymer plus the salt. Void-to-volume ratio is defined as the volume of the pores divided by the total volume of the polymeric layer including the volume of the pores. The void-to-volume ratio can be measured using the protocol described in AAMI (Association for the Advancement of Medical Instrumentation) VP20-1994, Cardiovascular ImplantsVascular Prosthesis section 8.2.1.2, Method for Gravimetric Determination of Porosity. The pores in the polymer can have an average pore diameter from about 1 micron to about 400 microns. Preferably the average pore diameter is from about 1 micron to about 100 microns, and more preferably is from about 1 micron to about 10 microns. The average pore diameter is measured based on images from a scanning electron microscope (SEM). Formation of porous THORALON is described, for example, in U.S. Pat. No. 6,752,826 and 2003/0149471 A1.

A variety of other biocompatible polyurethanes may be employed in the polymeric material(s) 18. These include polyurethane ureas that preferably include a soft segment and a hard segment formed from a diisocyanate and diamine. For example, polyurethane ureas with soft segments such as polytetramethylene oxide (PTMO), polyethylene oxide, polypropylene oxide, polycarbonate, polyolefin, polysiloxane (i.e. polydimethylsiloxane), and other polyether soft segments made from higher homologous series of diols may be used. Segments can be combined as copolymers or as blends. Mixtures of the soft segments may also be used. The soft segments also may have either alcohol end groups or amine end groups. The molecular weight of the soft segments may vary from about 500 to about 5,000 g/mole.

The diisocyanate may be represented by the formula OCN-R-NCO, where -R- may be aliphatic, aromatic, cycloaliphatic or a mixture of aliphatic and aromatic moieties. Examples of diisocyanates include MDI, tetramethylene diisocyanate, hexamethylene diisocyanate, trimethyhexamethylene diisocyanate, tetramethylxylylene diisocyanate, 4,4'-dicyclohexylmethane diisocyanate, dimer acid diisocyanate, isophorone diisocyanate, metaxylene diisocyanate, diethylbenzene diisocyanate, decamethylene 1,10 diisocyanate, cyclohexylene 1,2-diisocyanate, 2,4-toluene diisocyanate, 2,6-toluene diisocyanate, xylene diisocyanate, m-phenylene diisocyanate, hexahydrotolylene diisocyanate (and isomers), naphthylene-1,5-diisocyanate, 1-methoxyphenyl 2,4-diisocyanate, 4,4'-biphenylene diisocyanate, 3,3'-dimethoxy-4,4'-biphenyl diisocyanate and mixtures thereof.

The diamine used as a component of the hard segment includes aliphatic amines, aromatic amines and amines containing both aliphatic and aromatic moieties. For example, diamines include ethylene diamine, propane diamines, butanediamines, hexanediamines, pentane diamines, heptane diamines, octane diamines, m-xylylene diamine, 1,4-cyclohexane diamine, 2-methypentamethylene diamine, 4,4'-methylene dianiline, and mixtures thereof. The amines may also contain oxygen and/or halogen atoms in their structures.

The hard segment may be formed from one or more polyols. Polyols may be aliphatic, aromatic, cycloaliphatic or may contain a mixture of aliphatic and aromatic moieties. For example, the polyol may be ethylene glycol, diethylene glycol, triethylene glycol, 1,4-butanediol, 1,6-hexanediol, 1,8-octanediol, propylene glycols, 2,3-butylene glycol, dipropylene glycol, dibutylene glycol, glycerol, or mixtures thereof.

Biocompatible polyurethanes modified with cationic, anionic and aliphatic side chains may also be used. See, for example, U.S. Pat. No. 5,017,664.

Other biocompatible polyurethanes include: segmented polyurethanes, such as BIOSPAN; polycarbonate urethanes, such as BIONATE; and polyetherurethanes, such as ELASTHANE; (all available from POLYMER TECHNOLOGY GROUP, Berkeley, CA).

Other biocompatible polyurethanes include polyurethanes having a siloxane segment, also referred to as a siloxane-polyurethane. Examples of polyurethanes containing siloxane segments include polyether siloxane-polyurethanes, polycarbonate siloxane-polyurethanes, and siloxane-polyurethane ureas. Specifically, examples of siloxane-polyurethane include polymers such as ELAST-EON 2 and ELAST-EON 3 (AORTECH BIOMATERIALS, Victoria, Australia); polytetramethyleneoxide (PTMO) and polydimethylsiloxane (PDMS) polyether-based aromatic siloxane-polyurethanes such as PURSIL-10, -20, and -40 TSPU; PTMO and PDMS polyether-based aliphatic siloxane-polyurethanes such as PURSIL AL-5 and AL-10 TSPU; aliphatic, hydroxy-terminated polycarbonate and PDMS polycarbonate-based siloxane-polyurethanes such as CARBOSIL-10, -20, and -40 TSPU (all available from POLYMER TECHNOLOGY GROUP). The PURSIL, PURSIL -AL, and CARBOSIL polymers are thermoplastic elastomer urethane copolymers containing siloxane in the soft segment, and the percent siloxane in the copolymer is referred to in the grade name. For example, PURSIL-10 contains 10% siloxane. These polymers are synthesized through a multi-step bulk synthesis in which PDMS is incorporated into the polymer soft segment with PTMO (PURSIL) or an aliphatic hydroxy-terminated polycarbonate (CARBOSIL). The hard segment consists of the reaction product of an aromatic diisocyanate, MDI, with a low molecular weight glycol chain extender. In the case of PURSIL-AL the hard segment is synthesized from an aliphatic diisocyanate. The polymer chains are then terminated with a siloxane or other surface modifying end group. Siloxane-polyurethanes typically have a relatively low glass transition temperature, which provides for polymeric materials having increased flexibility relative to many conventional materials. In addition, the siloxane-polyurethane can exhibit high hydrolytic and oxidative stability, including improved resistance to environmental stress cracking. Examples of siloxane-polyurethanes are disclosed in U.S. Pat. Application Publication No. 2002/0187288 A1.

Biocompatible polyurethanes may be end-capped with surface active end groups, such as, for example, polydimethylsiloxane, fluoropolymers, polyolefin, polyethylene oxide, or other suitable groups. See, for example the surface active end groups disclosed in U.S. Pat. No. 5,589,563.

The porous polymeric sheet or polymeric coating may contain polytetrafluoroethylene or expanded polytetratfluoroethylene (ePTFE). Films or sheets of ePTFE are typically porous without the need for further processing. The structure of ePTFE can be characterized as containing nodes connected by fibrils. Porous ePTFE can be formed, for example, by blending PTFE with an organic lubricant and compressing it under relatively low pressure. Using a ram type extruder, the compressed polymer is then extruded through a die, and the lubricant is removed from the extruded polymer by drying or other extraction method. The dried material is then rapidly stretched and/or expanded at elevated temperatures. This process can provide for ePTFE having a microstructure characterized by elongated nodes interconnected by fibrils. Typically, the nodes are oriented with their elongated axis perpendicular to the direction of stretch. After stretching, the porous polymer is sintered by heating it to a temperature above its crystalline melting point while maintaining the material in its stretched condition. This can be considered as an amorphous locking process for permanently setting the microstructure in its expanded or stretched configuration. The structure and porosity of ePTFE is disclosed, for example, in U.S. Patent Nos. 6,547,815 B2; 5,980,799; and 3,953,566. Structures of porous hollow fibers can be formed from PTFE, and these porous hollow fibers can be assembled to provide a cohesive porous sheet or polymeric coating. Porous hollow fibers containing PTFE are disclosed, for example, in U.S. Patent No. 5,024,671.

Polymers can be processed to be porous sheets or coatings using standard processing methods, including solvent-based processes such as casting, spraying and dipping, and melt extrusion processes. Extractable pore or hole forming agents can be used during processing to produce porous sheets or coatings. Examples of extractable pore or hole forming agents include inorganic salts such as potassium chloride (KCl) and sodium chloride (NaCl), organic salts, and polymers such as poly(ethylene glycol) (PEG) and polyvinylpyrrolidone (PVP). Hole forming agents may have a particle size from about 10 µm to about 500 µm, from about 20 µm to about 100 µm, and from about 10 µm to about 40 µm. The amount of hole forming agent relative to the polymer may be from about 20 percent by weight (wt%) to about 90 wt%, and from about 40 wt% to about 70 wt%. These sizes and amounts of hole forming agents can provide for a high degree of porosity following extraction of the hole forming agent. The porosity can be from about 20 wt% to about 90 wt%, and from about 40 wt% to about 70 wt% of the final product.

Porous sheets or coatings may be in the form of a microporous, open-celled structure in which the holes are substantially interconnected. Microporous structures can be formed by extrusion of a mixture of polymer and one or more blowing agents. Microcellular polymeric foams can be produced by exposing the polymer to super-critical CO₂ under high temperature and pressure to saturate the polymer with the super-critical CO₂, and then cooling the polymer. Microcellular foams can be produced as described, for example, in U.S. Pat. Nos. 4,473,665 and 5,160,674. The foaming process can be carried out on extruded polymer tube by first dissolving an inert gas such as nitrogen or CO₂ under pressure into the polymer, and then forming microvoids by quickly decreasing the solubility of the gas in the polymer by changing the pressure or temperature, thus inducing thermodynamic instability. Examples of microporous polymeric structures are disclosed, for example, in U.S. Patent No. 6,702,849 B1.

The polymeric material 18 may be applied as a porous sheet or polymeric coating onto a surface of the vaso-occlusion device by any means known in the art, *e.g.*, dip coating, spray coating, wiping, vapor deposition or the like. The surface may be a metal, metal alloy, fibrous material or fabric. A polymeric coating disposed on the vaso-occlusion device may be made from a first polymer solution and a particulate, such as a salt, in a suitable solvent. The polymeric coating is not limited to any particular polymer, salt or solvent. The first polymer solution may one or variety of different polymers in a variety of different solvents. Further, a variety of different particulates or hole forming agents may be used as described above. As will be clear to those of skill in the art, the precise amounts of the polymer solution, solvent and particulate will vary, depending on the particular ingredients used. The particulate is added in the amount determined necessary to achieve the desired level of penetration into the substrate.

After applying the polymeric sheet or coating to a surface of the vaso-occlusion device, the coated vaso-occlusion device may be immersed in water or other suitable solvent to precipitate the polymer from the polymer solution and to allow holes to form according to a desired size, based on the amount of salt and polymer present. The vaso-occlusion device may alternatively contain one or more additional coatings, each differing in composition and/or chemical and physical properties. The additional coatings may be applied after drying the underlying coating(s) in an oven. The vaso-occlusion device may be partially, substantially or completely covered by the polymeric sheet or coating.

The polymeric material 18 may include a textile material. The textile includes fibers and may take many forms, including woven (including knitted) and non-woven. Preferably, the fibers of the textile comprise a synthetic polymer. Preferred textiles include those formed from polyethylene terephthalate and PTFE. These materials are inexpensive, easy to handle, have good physical characteristics and are suitable for clinical application.

Examples of biocompatible materials from which textiles can be formed include polyesters, such as poly(ethylene terephthalate); fluorinated polymers, such as polytetrafluoroethylene (PTFE) and fibers of expanded PTFE; and polyurethanes. In addition, materials that are not inherently biocompatible may be subjected to surface modifications in order to render the materials biocompatible. Examples of surface modifications include graft polymerization of biocompatible polymers from the material surface, coating of the surface with a crosslinked biocompatible polymer, chemical modification with biocompatible functional groups, and immobilization of a compatibilizing agent such as heparin or other substances. Thus, any fibrous material may be used to form a textile material, provided the final textile is biocompatible. Polymeric materials that can be formed into fibers suitable for making textiles include polyethylene, polypropylene, polyaramids, polyacrylonitrile, nylons and cellulose, in addition to polyesters, fluorinated polymers, and polyurethanes as listed above. Preferably the textile is made of one or more polymers that do not require treatment or modification to be biocompatible. More preferably, the textile is made of a biocompatible polyester. Examples of biocompatible polyesters include DACRON (DUPONT, Wilmington, DE) and TWILLWEAVE MICREL (VASCUTEK, Renfrewshire, Scotland).

Textile materials may be woven (including knitted) textiles or nonwoven textiles. Nonwoven textiles are fibrous webs that are held together through bonding of the individual fibers or filaments. The bonding can be accomplished through thermal or chemical treatments or through mechanically entangling the fibers or filaments. Because nonwovens are not subjected to weaving or knitting, the fibers can be used in a crude form without being converted into a yarn structure. Woven textiles are fibrous webs that have been formed by knitting or weaving. The woven textile structure may be any kind of weave including, for example, a plain weave, a herringbone weave, a satin weave, or a basket weave.

Woven fabrics may have any desirable shape, size, form and configuration. For example, the fibers of a woven fabric may be filled or unfilled. Examples of how the basic unfilled fibers may be manufactured and purchased are indicated in U.S. Pat. No. 3,772,137, by Tolliver. Fibers similar to those described are currently being manufactured by the DuPont Company from polyethylene terephthalate (often known as "DACRON" when manufactured by DuPont), and by other companies from various substances. Certain physical parameters may be used to characterize the textile fibers used in the polymeric material 18. The fibers may have a tensile strength of at least about 20,000 psi and a tensile modulus of at least about 2 × 10⁶ psi. Preferably, the textile is made of medical grade synthetic polymeric materials. The fibers of the textile may also have a high degree of axial orientation. The fibers may be of diameter from about 1 micron to about 5 millimeters. The denier of the textile may be from 0.5 denier per filament to 5 denier per filament. Preferably the interstices between the fibers of the textile comprise a maximum interstices spacing from about 1 micron to about 400 microns. More preferably, the interstices between the fibers of the textile comprise a maximum interstices spacing from about 1 micron to about 100 microns. Most preferably, the interstices between the fibers of the textile comprise a maximum interstices spacing from about 1 micron to about 10 microns.

The polymeric material 18 of the present invention may include any porous polymer; coating, textile or combination thereof set forth in co-pending Cook U.S. nonprovisional application no. 11/093,759, filed March 30, 2005.

The porous, polymeric material 18 may be impregnated with at least one bioactive agent. The bioactive agent may be incorporated into the holes 26 of the polymeric material 18 and/or chemically bonded to the polymer backbone using e.g., chemical cross-linking agents or other means conventionally available to those of skill in the art. The bioactive agent may be biochemical, organic, inorganic or synthetic in nature. Preferably the bioactive agent will be thrombogenic, fibrogenic, angiogenic, antithrombolytic, antifibrinolytic, fibrin stabilizing, wound healing, fibroblast stimulatory, vascularization promoting, cell and/or tissue attachment promoting, extracellular matrix promoting and/or the like. The bioactive agent may be a protein, peptide, growth factor, peptidomimetic, organic molecule, synthetic molecule, drug, synthetic polymer, or the like. Preferably, the bioactive agent will accelerate or support thrombosis, fibrosis, deposition of connective tissue (*e.g.*, collagen etc) in or around the vaso-occlusion device and/or stronger anchoring of the vaso-occlusion device to connective tissue.

In relation to the polymeric material 18 in which it is embedded, the bioactive agent may present in the polymeric material 18 in a range between about 0.005 % w/w and 50% w/w, between about 0.05 % and 10 % w/w, between about 0.1 % w/w and 2% w/w, between about 0.25% w/w and 1% w/w and combinations of ranges therefrom.

Exemplary biological bioactive agents include, but are not limited to, clotting factors, including, but not limited to plasmin, thrombin, prothrombin, fibrinogen, Factor V, Factor Va, Factor VII, Factor VIIa, Factor VIII, Factor VIIIa, Factor IX, Factor IXa, Factor X, Factor Xa, Factor XI, Factor XIa, Factor XII, XIIa, Factor XIII, von Willebrand Factor (vWF), other coagulation cascade factors and derivatives (*e.g.*, natural, synthetic, recombinant etc.) therefrom; antifibrinolytic agents, including, but not limited to, aminocaproic acid, aprotinin, tranexamic acid, desopressin, etamsylate; integrins; peptides containing RGD (arginine-glycine-aspartic acid) residues; cell attachment factors, including, but not limited to collagen (Types I-XIV), elastin, fibronectin, laminin, vitronectin; homocysteine; growth factors, including, but not limited to Connective Tissue Growth Factor (CTGF), Vascular Endothelial Growth Factor.(VEGF), Platelet Derived Growth Factor (PDGF), Fibroblast Growth Factor (FGF), Keratinocyte Growth Factor (KGF), Tumor Necrosis Factor (TNF), Epidermal Growth Factor (EGF), Transforming Growth Factor-alpha (TGF-α), Transforming Growth Factor-beta (TGF-β); cytokines, interleukins (*e.g*., IL-1, -2, -6, -8 *etc*.), chemokines having the above described chemical or biological properties. The polymeric material 18 may hold a single bioactive agent or a plurality of bioactive agents, as in the form of *e.g.,* a cocktail.

Resorbable agents 34 may be included in any part of the vaso-occlusion device to promote extracellular matrix deposition and bioremodeling, so as to stabilize and/or secure the vaso-occlusion device in a vessel site of a subject. For example, they may be incorporated into the holes 26 of the polymeric material 18 or disposed on the polymeric material 18 or any space-filling structural element surface. Alternatively, the resorbable agents 34 may be comprise some or all of the space-filling material used to expand an occlusion bag 38 and/or it may be included among the source materials from which the occlusion bag 38 is made.

Vaso-occlusive devices having resorbable agents 34, such as bioremodelable, extracellular matrix (ECM) materials, can allow for greater occlusion of the vessel and can help stabilize and/or secure the vaso-occlusion device in a vessel site of a subject by providing a matrix for promoting ECM deposition, remodeling, and growth of the surrounding tissue, including *e.g.*, promotion of cellular invasion, host incorporation, and absorption of the extracellular matrix material. As such, the resorbable agents 34 may facilitate stable resorption of the vaso-occlusion device in the body by at least partial replacement of the device by an individual's own tissue.

The ability to induce tissue remodeling may be ascribed to one or more bioactive agents (e.g., growth factor, etc.) in the resorbable agent 34 stimulating the infiltration of native cells into an acellular matrix, stimulating new blood vessel formation (capillaries) growing into the matrix to nourish the infiltrating cells (angiogenesis), and/or effecting the degradation and/or replacement of the bioremodelable material by endogenous tissue. Common events associated with bioremodeling may additionally include proliferation of granulation mesenchymal cells, biodegradation/resorption of implanted purified intestine submucosa material, and lack of immune rejection.

The submucosa or other ECM material used in the present invention may also exhibit an angiogenic character and thus be effective to induce angiogenesis in a host engrafted with the material. In this regard, angiogenesis is the process through which the body makes new blood vessels to generate increased blood supply to tissues. Thus, angiogenic materials, when contacted with host tissues, promote or encourage the infiltration of new blood vessels. Methods for measuring in vivo angiogenesis in response to biomaterial implantation have recently been developed. For example, one such method uses a subcutaneous implant model to determine the angiogenic character of a material. See, C. Heeschen et al., Nature Medicine 7 (2001), No. 7, 833-839. When combined with a fluorescence microangiography technique, this model can provide both quantitative and qualitative measures of angiogenesis into biomaterials. C. Johnson et al., Circulation Research 94 (2004), No. 2, 262-268.

Exemplary resorbable agents 34 include, but are not limited to, ECM materials, submucosal tissues, Type I to Type XIV collagens, fibrinogen, vitronectin, and combinations and/or derivatives therefrom. In a preferred embodiment, the resorbable agent 34 includes an ECM material having a native collagen containing tissue structure or purified collagen.

Resorbable agents 34 may include residual bioactive proteins or other ECM components derived from or native to the tissue source of the materials. Submucosa or ECM materials may include one or more growth factors, such as fibroblast growth factor 2 (FGF-2), vascular endothelial growth factor (VEGF), transforming growth factor- beta (TGF-beta), epidermal growth factor (EGF), platelet derived growth factor (PDGF), and/or isoforms or variants thereof. It is also expected that ECM base materials of the invention may contain additional bioactive components including, for example, one or more highly conserved collagens, growth factors, glycoproteins, proteoglycans, glycosaminoglycans, other growth factors, and other biological materials such as heparin, heparin sulfate, hyaluronic acid, fibronectin and the like. Thus, generally speaking, submucosal or other ECM materials may include a bioactive agent capable of inducing, directly or indirectly, a bioremodeling response reflected in a change in cell morphology, proliferation, growth, protein and/or gene expression. The bioactive agents in the ECM materials may be contained in their natural configuration and natural concentration.

Resorbable bioremodelable ECM materials may include highly conserved collagens, glycoproteins, proteoglycans, and glycosaminoglycans in their natural configuration and natural concentration. ECM materials may be isolated from warm-blooded vertebrate tissues including the alimentary, respiratory, intestinal, urinary or genital tracts of warm-blooded vertebrates. ECM materials may include submucosa, renal capsule membrane, dermal collagen, dura mater, pericardium, fascia lata, serosa, and peritnoneum or basement membrane layers, including liver basement membrane.

Submucosa or other ECM materials of the present invention can be derived from any suitable organ or other tissue source, usually sources containing connective tissues. The ECM materials processed for use in the invention will typically include abundant collagen, most commonly being constituted at least about 80% by weight collagen on a dry weight basis. Such naturally-derived ECM materials will for the most part include collagen fibers that are non-randomly oriented, for instance occurring as generally uniaxial or multi-axial but regularly oriented fibers. When processed to retain native bioactive factors, the ECM material can retain these factors interspersed as solids between, upon and/or within the collagen fibers. Particularly desirable naturally-derived ECM materials for use in the invention will include significant amounts of such interspersed, non-collagenous solids that are readily ascertainable under light microscopic examination with specific staining. Such non-collagenous solids can constitute a significant percentage of the dry weight of the ECM material in certain inventive embodiments, for example at least about 1 %, at least about 3%, and at least about 5% by weight in various embodiments of the invention.

A preferred ECM material is intestinal submucosa, particularly small intestinal submucosa (SIS). The preparation of intestinal submucosa (including SIS) is described and claimed in U.S. Pat. Nos. 6,206,931 and 6,358,284. A preferred intestinal submucosal tissue source in accordance with the present invention is pig SIS. Urinary bladder submucosa and its preparation are described in U.S. Pat. No. 5,554,389. Stomach submucosa and its preparation are described in U.S. Pat. No. 6,099,567.

ECM or submucosa materials may be sterilized and purified by a process involving delamination of disinfected submucosa tissue as described and claimed in U.S. Pat. Nos. 6,206,931 and 6,358,284. Alternatively, ECM or submucosa materials may be prepared by a process in which the sterilization step is carried out after delamination as described in U.S. Patent Nos. 5,993,844 and 6,572,650.

Submucosa or other ECM tissue used in the present invention is preferably highly purified, for example, as described in U.S. Patent No. 6,206,931 to Cook et al. Thus, preferred ECM material will exhibit an endotoxin level of less than about 12 endotoxin units (EU) per gram, more preferably less than about 5 EU per gram, and most preferably less than about 1 EU per gram. As additional preferences, the submucosa or other ECM material may have a bioburden of less than about 1 colony forming units (CFU) per gram, more preferably less than about 0.5 CFU per gram. Fungus levels are desirably similarly low, for example less than about 1 CFU per gram, more preferably less than about 0.5 CFU per gram. Nucleic acid levels are preferably less than about 5 µg/mg, more preferably less than about 2 µg/mg, and virus levels are preferably less than about 50 plaque forming units (PFU) per gram, more preferably less than about 5 PFU per gram. These and additional properties of submucosa or other ECM tissue taught in U.S. Patent No. 6,206,931 may be characteristic of the submucosa tissue used in the present invention.

ECM materials or tela submucosal tissues may be purified and processed into sheets, chunks, or alternatively, in fluidized or powdered forms. Fluidized or powdered forms of ECM/tela submucosa materials may be prepared using the techniques described in U.S. Pat. No. 6,206,931 or U.S. Pat. No. 5,275,826. The viscosity of fluidized submucosa compositions for use in accordance with this invention may be manipulated by controlling the concentration of the submucosa component and the degree of hydration. The viscosity may be adjusted to a range of about 2 to about 300,000 cps at 25°C. Higher viscosity formulations, for example, gels, may be prepared from the submucosa digest solutions by adjusting the pH of such solutions to about 6.0 to about 7.0.

ECM or tela submucosal materials may be optimally configured by stretching or by laminating together multiple pieces, layers or strips of tela submucosal tissue compressed under *e.g.*, dehydrating conditions in accordance with the teachings set forth in U.S. Pat. Nos. 6,206,931 and 6,358,284. As disclosed in the '931 and '284 patents, depending on the manner in which the pieces are overlayed together, multilaminate compositions may be engineered with different isotropic properties.

ECM or tela submucosal materials may serve as primary bioremodeling agents. Additional non-native bioactive components agents may be included in the ECM materials or in the polymeric materials 18 that cooperate in an additive or synergistic manner with the ECM materials to enhance remodeling of the surrounding tissue and/or anchoring of the vaso-occlusion device as described above. The non-native bioactive components, such as those synthetically produced by recombinant technology or other methods, may be incorporated into the submucosa or other ECM tissue. These non-native bioactive components may be naturally-derived or recombinantly produced proteins that correspond to those natively occurring in the ECM tissue, but perhaps of a different species (e.g. human proteins applied to collagenous ECMs from other animals, such as pigs). The non-native bioactive components may also be drug substances. Illustrative drug substances that may be incorporated into and/or onto the ECM materials used in the invention include, for example, antibiotics or thrombus-promoting substances such as blood clotting factors, e.g. thrombin, fibrinogen, and the like. These substances may be applied to the ECM material as a premanufactured step, immediately prior to the procedure (e.g. by soaking the material in a solution containing a suitable antibiotic such as cefazolin), or during or after engraftment of the material in the patient.

A thrombogenic fibrous material 30 may be incorporated into at least one surface of the vaso-occlusion device to promote embolization/thrombus formation. The thrombogenic fibrous material 30 may include synthetic and/or natural thrombogenic fibrous materials. Exemplary thrombogenic fibrous materials include, but are not limited to, Dacron, cotton, silk, wool, polyester thread and the like. Thrombogenic fibrous material 30 materials may be threaded, meshed, or braided. Preferably, the thrombogenic fibrous material 30 is physically or chemically associated with a surface of the vaso-occlusion device, including *e.g.*, the occlusion bag 38, space-filling structural elements 14, polymeric material 18 etc.

The vaso-occlusion device may further include radiopaque and/or MRI compatible marker materials to facilitate angiographic visualization. The radiopaque marker materials may be included within any aspect of the vaso-occlusion device and/or the porous, polymeric material 18. The radiopaque marker materials may be physically or chemically attached to any portion of the vaso-occlusion device. The marker materials may be part of *e.g.*, an occluding element 18, such as a coil, or they may be exogenously incorporated thereon or therein. They may be incorporated or introduced in liquid form, powdered form (*e.g.*, barium sulfate) or any other form suitable for rendering the occlusion device radiopaque.

Also described herein is a method for occluding a vessel in a patient is provided in which a vaso-occlusion device of the present invention is positioned in a vessel of a patient to occlude the vessel and/or promote thrombus formation. This method may involve a catheter assembly having a means for delivering a vaso-occlusion device of the present invention into a vessel of a patient, where the catheter assembly is used to position the vaso-occlusion device in a vessel to occlude the vessel. The catheter assembly provides a means for disengagement and release of the vaso-occlusion device to occlude the vessel.

Conventional catheters may be used to position and deliver the vaso-occlusion device in accordance with the present invention. The vaso-occlusion device may be delivered by any one of a variety of techniques employed for *e.g.*, the delivery of embolic coils, including those described in U.S. Pat. Nos. 4,994,069; 5,108,407; 5,122,136; 5,217,484; 5,226,911; 5,234,437; 5,250,071; 5,261,916; and 5,312,415. In some cases, it may be necessary to adapt or modify the construction of the vaso-occlusion device to be compatible with a known delivery system, *e.g.*, by including an anchor or a latch member at either or both ends of the vaso-occlusion device to facilitate delivery.

It is to be understood that the vaso-occlusion devices of the present invention are merely representative embodiments illustrating the principles of this invention and that other variations in the devices may be devised by those skilled in the art without departing from the scope of this invention. It is to be understood that this invention is not limited to the particular protocols, polymeric materials, bioactive agents described and as such may vary. Other uses of the vaso-occlusion device of this invention will be apparent to those of ordinary skill in the art. It is also to be understood that the terminology used herein is for the purpose of describing particular embodiments only, and is not intended to limit the scope of the present invention which will be limited only by the appended claims. It is therefore intended that the foregoing detailed description be regarded as illustrative rather than limiting, and that it be understood that it is the following claims, including all equivalents, that are intended to define the scope of this invention.

## Claims

1. An aneurysm occlusion device (10) comprising:
at least one occluding element (14);
at least one bioactive agent ; and
at least one polymeric material (18);
where at least a surface of the at least one occluding element is coated with the at least one polymeric material;
where the at least one bioactive agent promotes occlusion and where the device is configured to occlude an aneurysm in the subject; and
where the at least one occluding element is a space-filling structural element (14) which may assume any three-dimensional configuration sufficient for occluding the aneurysm;
where the at least one space-filling structural element is a coil, thread, or fiber, or combination thereof;
**characterized in that**
the at least one polymeric material has a plurality of holes (26) at least partially filled with the at least one bioactive agent.

2. The aneurysm occlusion device of claim 1, where at least one bioactive agent is a thrombogenic, fibrogenic, antithrombolytic or antifibrinolytic agent.

3. The aneurysm occlusion device of claim 2, where at least one bioactive agent is selected from the group consisting of plasmin, thrombin, prothrombin, fibrinogen, Factor V, Factor Va, Factor VII, Factor Vila, Factor VIII, Factor Villa, Factor IX, Factor IXa, Factor X, Factor Xa, Factor XI, Factor Xla, Factor Xii, XIIa, Factor XIII, vWF, aminocaproic acid, aprotinin, tranexamic acid, desopressin, etamsylate, integrin, peptide containing arginine-glycine-aspartic acid residues, collagen, elastin, fibronectin, laminin, vitronectin; homocysteine, CTGF, VEGF, PDGF, FGF, KGF, TNF, EGF, TGF-α, TGF-β, IL-1, IL-2, IL-6, and IL-8.

4. The aneurysm occlusion device of claim 1, further comprising a resorbable agent.

5. The aneurysm occlusion device of claim 1, where the at least one polymeric material comprises a polymer selected from the group consisting of polyesters, fluorinated polymers, polysiloxanes, polyurethanes, polyolefins, polyacrylonitrile, nylons, polyaramids and polysulfones.

6. The aneurysm occlusion device of claim 1, where the at least one polymeric material comprises a polyetherurethane urea and a surface modifying agent comprising a siloxane.

7. The aneurysm occlusion device of claim 1, where the at least one polymeric material comprises THORALON.

8. The aneurysm occlusion device of claim 1, where at least a surface of the occluding element comprises a thrombogenic fibrous material selected from the group consisting of DACRON, cotton, silk, wool, polyester, and combinations thereof.

## Patentansprüche

1. Aneurysmaverschlussvorrichtung (10), die Folgendes umfasst: zumindest ein Verschlusselement (14); zumindest ein biologisch wirksames Mittel; und zumindest ein Polymermaterial (18); worin zumindest eine Oberfläche des zumindest einen Verschlusselements mit dem zumindest einen Polymermaterial beschichtet ist; worin das zumindest eine biologisch wirksame Mittel den Verschluss fördert und worin die Vorrichtung zum Verschluss eines Aneurysmas in einer Person konfiguriert ist; und worin das zumindest eine Verschlusselement ein raumfüllendes Strukturelement (14) ist, das jede dreidimensionale Konfiguration annimmt, die zum Verschluss des Aneurysmas ausreicht; worin das zumindest eine raumfüllende Strukturelement eine Spule, ein Faden oder eine Faser oder ein Kombination davon ist; **dadurch gekennzeichnet, dass** das zumindest eine Polymermaterial eine Vielzahl von Löchern (26) aufweist, die zumindest teilweise mit dem zumindest einen biologisch wirksamen Mittel gefüllt sind.

2. Aneurysmaverschlussvorrichtung nach Anspruch 1, worin das zumindest eine biologisch wirksame Mittel ein thrombogenes, fibrogenes, antithrombolytisches oder antifibrinolytisches Mittel ist.

3. Aneurysmaverschlussvorrichtung nach Anspruch 2, worin das zumindest eine biologisch wirksame Mittel aus der aus Plasmin, Thrombin, Prothrombin, Fibrinogen, Faktor V, Faktor Va, Faktor VII, Faktor ViIa, Faktor ViIIa, Faktor IX, Faktor Ixa, Faktor X, Faktor Xa, Faktor XI, Faktor Xia, Faktor Xii, XIIa, Faktor XIII, vWF, Aminocapronsäure, Aprotinin, Tranexamsäure, Desopressin, Etamsylat, Integrin, peptidhaltigen Arginin-Glycin-Aspartsäure-Resten, Kollagen, Elastin, Fibronektin, Laminin, Vitronektin, Homocystein, CTGF, VEGF, PDGF, FGF, KGF, TNF, EGF, TGF-α, TGF-β, IL-I, IL-2, IL-6 und IL-8 bestehenden Gruppe ausgewählt ist.

4. Aneurysmaverschlussvorrichtung nach Anspruch 1, die ferner ein resorbierbares Mittel umfasst.

5. Aneurysmaverschlussvorrichtung nach Anspruch 1, worin das zumindest eine Polymermaterial ein aus der aus Polyester, fluorierte Polymere, Polysiloxane, Polyurethane, Polyolefine, Polyacrylnitril, Nylon, Polyaramide und Polysulfone bestehenden Gruppe ausgewähltes Polymer umfasst.

6. Aneurysmaverschlussvorrichtung nach Anspruch 1, worin das zumindest eine Polymermaterial einen Polyetherurethan-Harnstoff und ein Siloxan umfassendes oberflächenmodifizierendes Mittel umfasst.

7. Aneurysmaverschlussvorrichtung nach Anspruch 1, worin das zumindest eine Polymermaterial THORALON umfasst.

8. Aneurysmaverschlussvorrichtung nach Anspruch 1, worin zumindest eine Oberfläche des Verschlusselements ein thrombogenes Fasermaterial umfasst, das aus der aus DACRON, Baumwolle, Seide, Wolle, Polyester und Kombinationen davon bestehenden Gruppe ausgewählt ist.

## Revendications

1. Dispositif d'occlusion d'anévrisme (10), comprenant:
au moins un élément d'occlusion (14);
au moins un agent bio-actif; et
au moins une matière polymère (18),
dans lequel au moins une surface dudit au moins un élément d'occlusion est revêtue avec ladite au moins une matière polymère;
dans lequel ledit au moins un agent bio-actif favorise l'occlusion, et dans lequel le dispositif est configuré de manière à occlure un anévrisme chez le sujet; et
dans lequel ledit au moins un élément d'occlusion est un élément structural de remplissage d'espace (14) qui est capable d'adopter n'importe quelle configuration en trois dimensions suffisante pour occlure l'anévrisme;
dans lequel ledit au moins un élément structural de remplissage d'espace est une bobine, un fil ou une fibre, ou une combinaison de celles-ci,
**caractérisé en ce que** ladite au moins une matière polymère comporte une pluralité de trous (26) qui sont au moins partiellement remplis avec ledit au moins un agent bio-actif.

2. Dispositif d'occlusion d'anévrisme selon la revendication 1, dans lequel ledit au moins un agent bio-actif est un agent thrombogène, fibrogène, anti-thrombolytique ou anti-fibrinolytique.

3. Dispositif d'occlusion d'anévrisme selon la revendication 2, dans lequel au moins un agent bio-actif est sélectionné dans le groupe comprenant la plasmine, la thrombine, la prothrombine, le fibrinogène, le Facteur V, le Facteur Va, le Facteur VII, le Facteur ViIa, le Facteur VIII, le Facteur ViIIa, le Facteur IX, le Facteur IXa, le Facteur X, le Facteur Xa, le Facteur XI, le Facteur XIa, le Facteur Xii, le XIIa, le Facteur XIII, le vWF, l'acide aminocaproïque, l'aprotinine, l'acide tranexamique, le desopressine, l'étamsylate, l'intégrine, des résidus d'acide arginine-glycine-aspartique contenant des peptides, le collagène, l'élastine, la fibronectine, la laminine, la vitronectine; l'homocystéine, le CTGF, le VEGF, le PDGF, le FGF, le KGF, le TNF, l'EGF, le TGF-α, le TGF-β, l'IL-1, l'IL-2, l'IL-6 et l'IL-8.

4. Dispositif d'occlusion d'anévrisme selon la revendication 1, comprenant en outre un agent résorbable.

5. Dispositif d'occlusion d'anévrisme selon la revendication 1, dans lequel au moins une matière polymère comprend un polymère qui est sélectionné dans le groupe composé des polyesters, des polymères fluorés, des polysiloxanes, des polyuréthanes, des polyoléfines, du polyacrylonitrile, des nylons, des polyaramides et des polysulfones.

6. Dispositif d'occlusion d'anévrisme selon la revendication 1, dans lequel ladite au moins une matière polymère comprenant une polyétheruréthane urée et un agent de modification de surface qui contient un siloxane.

7. Dispositif d'occlusion d'anévrisme selon la revendication 1, dans lequel ladite au moins une matière polymère comprend le THORALON.

8. Dispositif d'occlusion d'anévrisme selon la revendication 1, dans lequel au moins une surface de l'élément d'occlusion comprend une matière fibreuse thrombogène qui est sélectionnée dans le groupe comprenant le DACRON, le coton, la soie, la laine, le polyester et des combinaisons de ceux-ci.
